# EUROPEAN PATENT APPLICATION

(11) **EP 3 249 270 A1**
(43) Date of publication of application: **29.11.2017**
(21) Application number: 16875147.7
(22) Date of filing: 29.07.2016
(51) Int. Cl.: F16K 7/06, A61M 5/142, F04B 43/12, F04C 5/00, F16K 11/02

(54) **FLUID SWITCHING VALVE, FLUID TRANSPORT CARTRIDGE, AND FLUID TRANSPORT DRIVE UNIT**

(30) Priority: 15.12.2015 JP 2015244579
(71) Applicant: Primetech Corporation, Bunkyo-ku, Tokyo 112-0002 (JP)
(72) Inventor: OGIHARA Ryosuke, Ichikawa-shi Chiba 272-0804 (JP)
(74) Representative: Pfenning, Meinig & Partner mbB
(86) International application number: PCT/JP2016/072293
(87) International publication number: WO 2017/104160

(57) **Abstract**

There is disclosed a fluid switching valve that can switch fluid path states among a plurality of fluid paths by a simple configuration. A fluid switching valve including a first elastic tube that delivers a first fluid; a second elastic tube that delivers a second fluid; a third elastic tube having one end connected to the first and second elastic tubes; and a switching member that, by displacing a protruding portion, switches between a first state in which the first elastic tube is constricted and the second fluid is delivered to the third elastic tube, a second state in which the second elastic tube is constricted and the first fluid is delivered to the third elastic tube, a third state in which the first and second elastic tubes are both constricted and introduction of fluid to the third elastic tube is cut off, and a fourth state in which neither the first nor second elastic tube is constricted and both the first and second fluids are delivered to the third elastic tube.

## Description

### BACKGROUND

### 1. TECHNICAL FIELD

The present invention relates to a fluid switching valve, a fluid delivery cartridge, and a fluid delivery driving unit.

### 2. RELATED ART

A fluid switching valve is known that can displace one switching member relative to a plurality of fluid paths in order to switch between a portion of fluid path states among all possible fluid path states, as shown in Patent Document 1, for example.
Patent Document 1: Japanese Patent Application Publication No. 2013-44411

There is a demand for a fluid switching valve that can arbitrarily switch between all fluid path states, which include combinations of closed and open fluid paths among a plurality of fluid paths, by displacing one switching member.

### SUMMARY

According to a first aspect of the present invention, provided is a fluid switching valve comprising a first elastic tube that delivers a first fluid; a second elastic tube that delivers a second fluid; a third elastic tube having one end connected to the first elastic tube and the second elastic tube; and a switching member that, by displacing a protruding portion, switches between a first state in which the first elastic tube is constricted and the second fluid is delivered to the third elastic tube, a second state in which the second elastic tube is constricted and the first fluid is delivered to the third elastic tube, a third state in which the first elastic tube and the second elastic tube are both constricted and introduction of fluid to the third elastic tube is cut off, and a fourth state in which neither the first elastic tube nor the second elastic tube is constricted and both the first fluid and the second fluid are delivered to the third elastic tube.

According to a second aspect of the present invention, provided is a fluid delivery cartridge comprising a fluid switching valve; a first supply port that supplies the first fluid to the first elastic tube; a second supply port that supplies the second fluid to the second elastic tube; and a discharge port that discharges the first fluid and the second fluid from the third elastic tube.

According to a third aspect of the present invention, provided is a fluid delivery driving unit for driving a fluid delivery cartridge in which are housed a first elastic tube that delivers a first fluid, a second elastic tube that delivers a second fluid, and a third elastic tube having one end connected to the first elastic tube and the second elastic tube, the fluid delivery driving unit comprising a mounting portion on which the fluid delivery cartridge is mounted; a switching member that includes a protruding portion that faces at least one of the first elastic tube and the second elastic tube when the fluid delivery cartridge is mounted on the mounting portion; and a driving section that displaces the switching member, wherein the switching member, as a result of the protruding portion moving due to displacement by the driving section, switches between a first state in which the first constricting lever is pressed, the first elastic tube is constricted, and the second fluid is delivered to the third elastic tube; a second state in which the second constricting lever is pressed, the second elastic tube is constricted, and the first fluid is delivered to the third elastic tube; a third state in which the first constricting lever and the second constricting lever are pressed, the first elastic tube and the second elastic tube are both constricted, and introduction of fluid to the third elastic tube is cut off; and a fourth state in which neither the first elastic tube nor the second elastic tube is constricted and both the first fluid and the second fluid are delivered to the third elastic tube.

The summary clause does not necessarily describe all necessary features of the embodiments of the present invention. The present invention may also be a subcombination of the features described above.

### BRIEF DESCRIPTION OF THE DRAWINGS

Fig. 1 shows one example of a usage state of a fluid delivery apparatus according to the present embodiment.
Fig. 2 is a top surface view of the fluid delivery apparatus.
Fig. 3 is a schematic view of the internal structure of the fluid delivery cartridge.
Fig. 4A is a cross-sectional view taken along the line A-A shown in Fig. 3.
Fig. 4B is a cross-sectional view taken along the line B-B shown in Fig. 4A.
Fig. 5A is a schematic view describing the switching of the flow paths in the fluid switching valve.
Fig. 5B is a schematic view describing the switching of the flow paths in the fluid switching valve.
Fig. 5C is a schematic view describing the switching of the flow paths in the fluid switching valve.
Fig. 5D is a schematic view describing the switching of the flow paths in the fluid switching valve.
Fig. 6 is a schematic view for describing a protrusion formed on an end portion of a constricting lever.
Fig. 7A is a schematic view for describing a constricting lever according to a second embodiment.
Fig. 7B is a schematic view for describing a constricting lever according to a second embodiment.
Fig. 7C is a schematic view for describing a constricting lever according to a second embodiment.
Fig. 8A is a schematic view for describing a constricting mechanism according to a third embodiment.
Fig. 8B is a schematic view for describing a constricting mechanism according to a third embodiment.
Fig. 8C is a schematic view for describing a constricting mechanism according to a third embodiment.
Fig. 8D is a schematic view for describing a constricting mechanism according to a third embodiment.
Fig. 9A is a schematic view for describing a fluid switching valve according to a fourth embodiment.
Fig. 9B is a schematic view for describing a fluid switching valve according to a fourth embodiment.
Fig. 10 is a perspective view for describing a fluid delivery driving unit according to a fifth embodiment.
Fig. 11 is a schematic view of the internal configuration of a fluid delivery cartridge according to a fifth embodiment.
Fig. 12 is a cross-sectional view taken along the line C-C shown in Fig. 10.

### (General Disclosure)

A fluid switching valve may comprise a first elastic tube that delivers a first fluid, a second elastic tube that delivers a second fluid, and a third elastic tube having one end connected to the first elastic tube and the second elastic tube. The fluid switching valve may comprise a switching member that, by displacing a protruding portion, switches between a first state in which the first elastic tube is constricted and the second fluid is delivered to the third elastic tube, a second state in which the second elastic tube is constricted and the first fluid is delivered to the third elastic tube, a third state in which the first elastic tube and the second elastic tube are both constricted and introduction of fluid to the third elastic tube is cut off, and a fourth state in which neither the first elastic tube nor the second elastic tube is constricted and both the first fluid and the second fluid are delivered to the third elastic tube.

The fluid switching valve may comprise a first constricting lever that constricts the first elastic tube, and a second constricting lever that constricts the second elastic tube. The protruding portion may include a first protruding portion that presses the first constricting lever to constrict the first elastic tube and a second protruding portion that presses the second constricting lever to constrict the second elastic tube.

The switching member may slidingly move in a direction orthogonal to a direction in which the first constricting lever and the second constricting lever press the first elastic tube and the second elastic tube.

At least one of the first constricting lever and the second constricting lever may include a guide portion for guiding a tip portion thereof toward an elastic tube to be constricted.

At least one of the first constricting lever and the second constricting lever that is provided with the guide portion may be supported slidably in one direction.

The first constricting lever may include a protrusion that has a smaller contact surface area on a contact surface thereof contacting the first elastic tube.

The fluid switching valve may be provided with a stepped portion on a contact surface of at least one of the first constricting lever and the first protruding portion. In the fluid switching valve, a constriction amount by which the first constricting lever constricts the first elastic tube may be able to be adjusted in a stepped manner.

The fluid switching valve may comprise a first constricting cam that constricts the first elastic tube, and a second constricting cam that constricts the second elastic tube. The protruding portion may include a first protruding portion that rotates the first constricting cam to constrict the first elastic tube and a second protruding portion that rotates the second constricting cam to constrict the second elastic tube.

The switching member may slidingly move in a direction orthogonal to a direction in which the first constricting cam and the second constricting cam press the first elastic tube and the second elastic tube.

The switching member may be a rotating member that moves rotationally, and the protruding portion may include a first protruding portion that is formed partially protruding in a radial direction centered on a rotational axis and constricts the first elastic tube, and a second protruding portion that constricts the second elastic tube.

The switching member may perform switching in order of the fourth state, the first state, the third state, the second state, and the fourth state, or in order of the fourth state, the second state, the third state, the first state, and the fourth state.

The fluid switching valve may comprise a fourth elastic tube that has one end connected to the third elastic tube and delivers a third fluid, and a third constricting cam that constricts the fourth elastic tube. The switching member may be T-shaped and control delivery of the first fluid, the second fluid, and the third fluid by slidingly moving in two directions.

The fluid switching valve may comprise a fifth elastic tube that has one end connected to the third elastic tube and delivers a fourth fluid, and a fourth constricting cam that constricts the fifth elastic tube. The switching member may be cross-shaped and control delivery of the first fluid, the second fluid, the third fluid, and the fourth fluid by slidingly moving in two directions.

A fluid delivery cartridge may comprise any one of the fluid switching valves described above, a first supply port that supplies the first fluid to the first elastic tube, a second supply port that supplies the second fluid to the second elastic tube, and a discharge port that discharges the first fluid and the second fluid from the third elastic tube.

In the fluid delivery cartridge, in an initial state before use, the fluid switching valve is fixed to maintain the fourth state.

The fluid delivery cartridge may comprise a transfer mechanism that transfers drive force generated by an external device to displace the switching member.

The fluid delivery cartridge may comprise at least one of a drive mechanism that peristaltically moves the third elastic tube to move fluid therein to the discharge port and a driving section that displaces the switching member.

The fluid delivery cartridge may comprise a first elastic tube that delivers a first fluid and a second elastic tube that delivers a second fluid. In the fluid delivery cartridge, a third elastic tube having one end connected to the first elastic tube and the second elastic tube may be housed. A fluid delivery driving unit may comprise a mounting portion on which a fluid delivery cartridge is mounted, a switching member that includes a protruding portion that faces at least one of the first elastic tube and the second elastic tube when the fluid delivery cartridge is mounted on the mounting portion, and a driving section that displaces the switching member. The switching member, by being displaced by the driving section to move the protruding portion, may switch the fluid delivery cartridge between a first state in which the first elastic tube is constricted and the second fluid is delivered to the third elastic tube; a second state in which the second elastic tube is constricted and the first fluid is delivered to the third elastic tube; a third state in which the first elastic tube and the second elastic tube are both constricted and introduction of fluid to the third elastic tube is cut off; and a fourth state in which neither the first elastic tube nor the second elastic tube is constricted and both the first fluid and the second fluid are delivered to the third elastic tube.

The fluid delivery driving unit may comprise a restricting member that restricts reaction of the protruding portion occurring when at least one of the first elastic tube and the second elastic tube is constricted.

The first restricting lever and the second restricting lever may be supported in a manner that enables sliding in a direction parallel to a movement direction of the switching member, at a body portion that extends from a portion pressed by the protruding portion.

The body portion of the first constricting lever may include an escaping portion that crosses over the first elastic tube in a direction orthogonal to a sliding direction. The body portion of the second constricting lever may include an escaping portion that crosses over the second elastic tube in a direction orthogonal to the sliding direction.

### DESCRIPTION OF EXEMPLARY EMBODIMENTS

Hereinafter, some embodiments of the present invention will be described. The embodiments do not limit the invention according to the claims, and all the combinations of the features described in the embodiments are not necessarily essential to means provided by aspects of the invention.

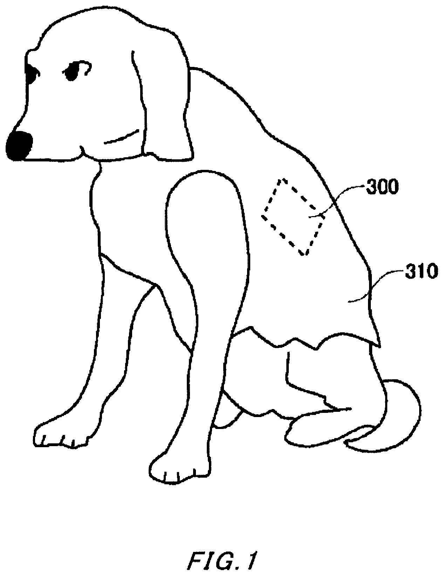

Fig. 1 shows one example of a usage state of a fluid delivery apparatus 300 according to the present embodiment. The fluid delivery apparatus 300 is secured to test clothing 310 worn by a test animal, e.g. a dog or monkey, serving as a test subject. The fluid delivery apparatus 300 injects fluid such as medicine into the body of the test animal, in units of a discharge amount determined according to predetermined setting conditions. The setting conditions are programmed to include conditions such as discharge start time, a discharge ratio indicating the discharge amount per unit time, discharge duration, and discharge interval. If necessary, the setting conditions can be changed by remote operation via wireless communication.

The fluid is not limited to fluids such as medicinal fluid, saline solution, and nutrient solution, and may be gas or gel that includes the component to be discharged. The fluid delivery apparatus 300 has a size that enables a medium-sized pet or test animal to move while wearing the fluid delivery apparatus 300, and therefore the subject wearing the fluid delivery apparatus 300 is not limited to a test animal, and can be a human instead. By having a human wear the fluid delivery apparatus 300, medicinal fluid can be provided periodically or at planned times to living tissue, such as blood vessels or muscles, for example.

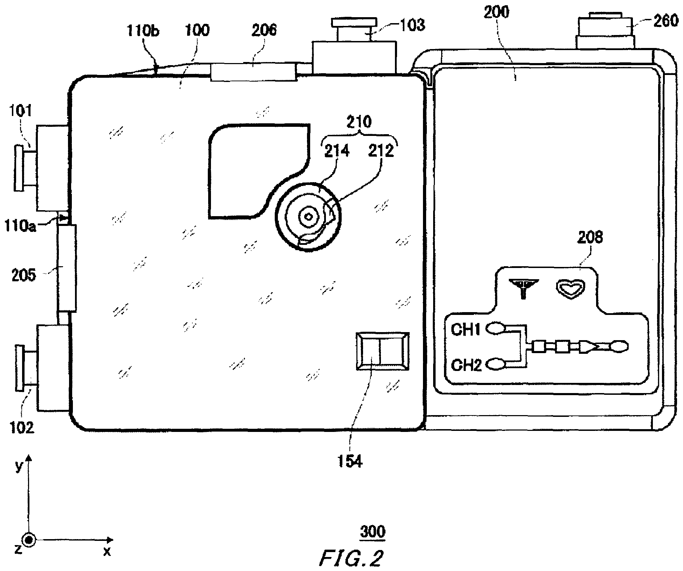

Fig. 2 is a top surface view of the fluid delivery apparatus 300. The fluid delivery apparatus 300 includes a fluid delivery cartridge 100 and a fluid delivery driving unit 200. The fluid delivery apparatus 300 can selectively discharge two types of fluid. The fluid delivery cartridge 100 is fixed in a detachable manner to the fluid delivery driving unit 200. The fluid delivery cartridge 100 includes a first fluid inflow portion 101, a second fluid inflow portion 102, and a fluid discharge portion 103. The first fluid inflow portion 101 is connected to an external first reservoir that contains a first fluid. The second fluid inflow portion 102 is connected to a second reservoir that contains a second fluid. The fluid discharge portion 103 is connected to an inflow tube for injecting the first fluid and the second fluid into the body of the test animal.

The fluid delivery driving unit 200 includes a cam unit 210. The cam unit 210 includes a cam body 212 that is rotationally driven and a cam cover 214 that houses the cam body 212. The cam unit 210 is a drive mechanism that guides the first fluid or the second fluid from the first fluid inflow portion 101 or the second fluid inflow portion 102 to the fluid discharge portion 103. The cam unit 210 of the fluid delivery driving unit 200 also functions to fix the position of the fluid delivery cartridge 100. The cam cover 214 of the cam unit 210 is shaped as an arc around a rotational center, and when the fluid delivery cartridge 100 is to be fixed, the cam cover 214 sets the position of the fluid delivery cartridge 100 relative to the fluid delivery driving unit 200.

The fluid delivery driving unit 200 includes a LED display section 208. The LED display section 208 displays the fluid path state or the like in the fluid delivery cartridge 100, which is described further below. For example, when the first fluid is in a state of being delivered to the fluid discharge portion 103, the LED corresponding to "CH1" shown in Fig. 2 lights up. Furthermore, when the second fluid is in a state of being delivered to the fluid discharge portion 103, the LED corresponding to "CH2" shown in Fig. 2 lights up.

Furthermore, the fluid delivery driving unit 200 includes an input terminal 260 for acquiring biometric information relating to the test animal. The biometric information is information relating to an electrocardiogram or information relating to the blood pressure of the test animal, for example. The biometric information may be used to control discharge of the fluid. If the discharge of the fluid is not controlled according to the biometric information, the input terminal 260 need not be provided.

The fluid delivery driving unit 200 includes a first hook 205 and a second hook 206 for fixing the fluid delivery cartridge 100. The first hook 205 fixes an edge 110a of the fluid delivery cartridge 100 between the first fluid inflow portion 101 and the second fluid inflow portion 102. The second hook 206 fixes an edge 110b of the fluid delivery cartridge 100 where the fluid discharge portion 103 is provided. The fluid delivery cartridge 100 and the fluid delivery driving unit 200 are fixed by the locking member 154. In this way, by fixing the fluid delivery cartridge 100 on two edges together with the locking member 154, the fluid delivery cartridge 100 is prevented from falling off of the fluid delivery driving unit 200.

Furthermore, when the fluid delivery cartridge 100 is to be removed, the locking member 154 is released and the first hook 205 is pressed by the thumb of the right hand, for example, while pressing the second hook 206 with another finger of the right hand, and the fluid delivery cartridge 100 can then be removed by the left hand. Accordingly, the fluid delivery cartridge 100 can be easily detached from the fluid delivery driving unit 200. In this way, by including different fixing functions such as the first hook 205, the second hook 206, and the locking member 154 in the fluid delivery apparatus 300, when the fluid delivery apparatus 300 is worn by a primate such as a monkey, the fluid delivery cartridge 100 can be prevented from falling off as a result of the primate touching the fluid delivery cartridge 100.

The coordinate axes are determined as shown in Fig. 2. Specifically, a direction that is parallel to the rotating shaft of the cam body 212 and pointing toward the top of the drawing is determined to be the positive Z-axis direction, a direction that is orthogonal to the Z axis and parallel to the edge 110a is determined to be the Y axis, and a direction orthogonal to the Z axis and parallel to the edge 110b is the X axis. The X-axis and Y-axis directions are defined in a right-handed system. Furthermore, in the following description, the positive Z-axis direction is defined as being upward, the negative Z-axis direction is defined as being downward, the positive Y-axis direction is defined as being to the right, and the negative Y-axis direction is defined as being to the left. In several of the following drawings, the coordinate axes of Fig. 2 are used as a reference to display the coordinate axes in a manner to understand the orientation of each drawing.

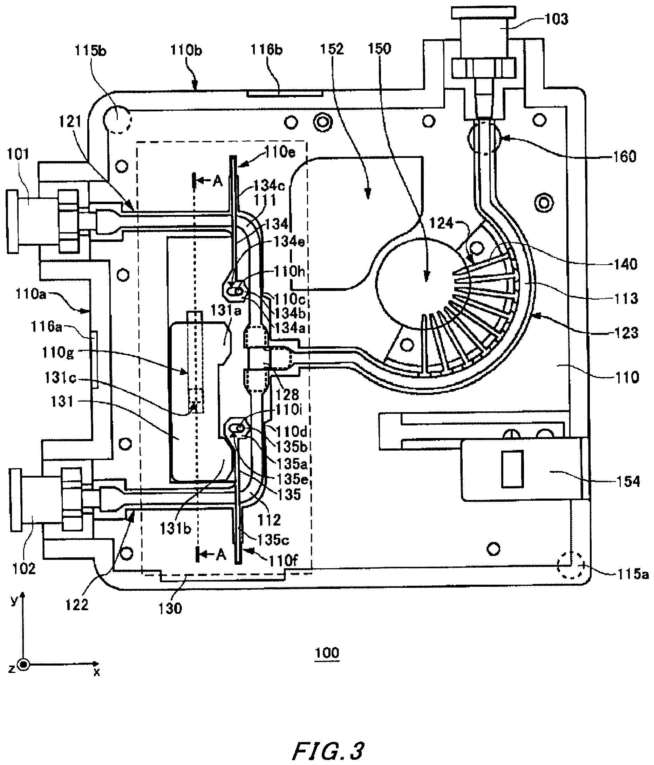

Fig. 3 is a schematic view of the internal structure of the fluid delivery cartridge 100. The fluid delivery cartridge 100 includes an external packaging member 110, a fluid switching valve 130, and a plurality of pressing pins 140. The external packaging member 110 houses the fluid switching valve 130 and the plurality of pressing pins 140. In the external packaging member 110, at least the outward-facing surface that is opposite the inward-facing surface facing the fluid delivery driving unit 200 is transparent. The locking member 154 for fixing the fluid delivery cartridge 100 to the fluid delivery driving unit 200 is provided in the external packaging member 110.

The contour of the external packaging member 110 as seen from the outward-facing surface or inward-facing surface of the fluid delivery cartridge 100 is substantially rectangular, in the portion not including the first fluid inflow portion 101, the second fluid inflow portion 102, and the fluid discharge portion 103, and is fixed to the fluid delivery driving unit 200 at two edges of the rectangular contour. These two edges are the edge 110a, which is provided with the first fluid inflow portion 101 and the second fluid inflow portion 102 and also includes the portion between the first fluid inflow portion 101 and the second fluid inflow portion 102, and the edge 110b, which is provided with the fluid discharge portion 103.

The external packaging member 110 includes a first fastening portion 116a to which the first hook 205 is fixed, on the edge 110a. The external packaging member 110 includes a second fastening portion 116b to which the second hook 206 is fixed, on the edge 110b.

The fluid switching valve 130 includes a first elastic tube 111, a second elastic tube 112, a third elastic tube 113, and a three-way branch member 128. Furthermore, the fluid switching valve 130 includes a switching member 131, as well as a constricting lever 134 and a constricting lever 135.

The first elastic tube 111 is arranged along a first groove 121 formed in the external packaging member 110, and forms a first fluid path. One end of the first elastic tube 111 is connected to the first fluid inflow portion 101, and the other end is connected to the three-way branch member 128. The first elastic tube 111 delivers the first fluid introduced through the first fluid inflow portion 101 from the first reservoir to the three-way branch member 128.

The second elastic tube 112 is arranged along a second groove 122 formed in the external packaging member 110, and forms a second fluid path. One end of the second elastic tube 112 is connected to the second fluid inflow portion 102, and the other end is connected to the three-way branch member 128. The second elastic tube 112 delivers the second fluid introduced through the second fluid inflow portion 102 from the second reservoir to the three-way branch member 128.

The third elastic tube 113 is arranged along a third groove 123 formed in the external packaging member 110, and forms a third fluid path. One end of the third elastic tube 113 is connected to the first elastic tube 111 and the second elastic tube 112, via the three-way branch member 128, and the other end is connected to the fluid discharge portion 103. The third elastic tube 113 delivers at least one of the first fluid and the second fluid introduced through the three-way branch member 128 to the fluid discharge portion 103. The first fluid inflow portion 101, the second fluid inflow portion 102, and the fluid discharge portion 103 may be Luer-Lok style connectors, which are easily detachable.

The fluid switching valve 130 is a mechanism for switching between four fluid path states. The first state is a fluid path state in which the first fluid path is closed, the second fluid path is open, and the second fluid is guided to the fluid discharge portion 103. The second state is a fluid path state in which the first fluid path is open, the second fluid path is closed, and the first fluid is guided to the fluid discharge portion 103. The third state is a fluid path state in which the first fluid path and the second fluid path are both closed, and neither the first fluid nor the second fluid is guided to the fluid discharge portion 103. The fourth state is a fluid path state in which the first fluid path and the second fluid path are both open, and both the first fluid and the second fluid are guided to the fluid discharge portion 103.

Fig. 3 shows the fourth state, in which the first fluid path and the second fluid path are both open. In the fluid switching valve 130, the switching of the fluid being introduced into the third elastic tube 113 is realized by the operation of the switching member 131, the constricting lever 134, and the constricting lever 135.

The switching member 131 includes a protruding portion 131 a and a protruding portion 131b. The constricting lever 134 includes a pressing member 134a and a body portion 134c, and the constricting lever 135 includes a pressing member 135a and a body portion 135c. The pressing member 134a includes a protrusion 134b, and the pressing member 135a includes a protrusion 135b. The constricting lever 134 is arranged such that the pressing member 134a faces the first elastic tube 111, in order to press the first elastic tube 111. The constricting lever 135 is arranged such that the pressing member 135a faces the second elastic tube 112, in order to press the second elastic tube 112.

The switching member 131 is arranged facing the first elastic tube 111 and the second elastic tube 112, and sandwiches at least one of the constricting lever 134 and the constricting lever 135. A constriction assisting member 110c for assisting with the constriction effect of the first elastic tube 111 by the pressing member 134a is formed in the external packaging member 110 at a position facing the pressing member 134a, such that the first elastic tube 111 is sandwiched therebetween. Similarly, a constriction assisting member 110d for assisting with the constriction effect of the second elastic tube 112 by the pressing member 135a is formed in the external packaging member 110 at a position facing the pressing member 135a, such that the second elastic tube 112 is sandwiched therebetween.

Furthermore, a restricting pin 110h is formed by monolithic molding in the external packaging member 110 near the position where the constricting lever 134 constricts the first elastic tube 111. Similarly, a restricting pin 110i is formed by monolithic molding in the external packaging member 110 near the position where the constricting lever 135 constricts the second elastic tube 112. The restricting pins 110h and 110i respectively restrict displacement of the pressing members 134a and 135a in the Y direction.

The end of the constricting lever 134 that is opposite the end on which the pressing member 134a is formed is slidably inserted in the Y-axis direction into the supporting portion 110e of the external packaging member 110. A body portion 134c, which is the portion of the constricting lever 134 other than the pressing member 134a, is an elastic plate. The body portion 134c includes an escaping portion that crosses over the first elastic tube 111 in the Z direction.

The constricting lever 134 is provided with a guide hole 134e on the end where the pressing member 134a is formed. The guide hole 134e is a hole which penetrates through the pressing member 134a in the Z-axis direction. The XY cross section of the guide hole 134e has a substantially elliptical shape that is long in the X direction. Furthermore, the width of the XY cross section of the guide hole 134e in the Y direction is substantially the same as the diameter of the restricting pin 110h. Here, the guide hole 134e plays the role of a guide portion for guiding the pressing member 134a, which is the tip portion of the constricting lever 134. The restricting pin 110h is inserted into the guide hole 134e. The pressing member 134a can be restricted from being displaced in the Y-axis direction by the restricting pin 110h, while being allowed to be displaced in the X-axis direction, which is the direction for constricting the first elastic tube 111.

The end of the constricting lever 135 that is opposite the end on which the pressing member 135a is formed is slidably inserted in the Y-axis direction into the supporting portion 110f of the external packaging member 110. A body portion 135c, which is the portion of the constricting lever 135 other than the pressing member 135a, is an elastic plate. The body portion 135c includes an escaping portion that crosses over the second elastic tube 112 in the Z direction.

The constricting lever 135 is provided with a guide hole 135e on the end where the pressing member 135a is formed. The guide hole 135e is a hole which penetrates through the pressing member 135a in the Z-axis direction. The XY cross section of the guide hole 135e has a substantially elliptical shape that is long in the X direction. Furthermore, the width of the XY cross section of the guide hole 135e in the Y direction is substantially the same as the diameter of the restricting pin 110i. Here, the guide hole 135e plays the role of a guide portion for guiding the pressing member 135a, which is the tip portion of the constricting lever 135. The restricting pin 110i is inserted into the guide hole 135e. The pressing member 135a can be restricted from being displaced in the Y-axis direction by the restricting pin 110i, while being allowed to be displaced in the X-axis direction, which is the direction for constricting the second elastic tube 112.

The switching member 131 is driven by the drive apparatus described further below, to move in the Y direction. The switching member 131 includes an insertion hole 131c on a mounting surface side that faces the fluid delivery driving unit 200. A notched groove 110g is formed in the surface of the external packaging member 110 facing the switching member 131. As described in detail further below, a fixing member, which is a portion of the drive apparatus exposed in the mounting surface of the fluid delivery driving unit 200, is inserted into the insertion hole 131c via a notched groove 110g. In this way, the switching member 131 is locked to a rack member of the drive apparatus, and moves in the Y direction in conjunction with the rack member.

In accordance with the movement of the switching member 131, the protruding portion 131 a and the protruding portion 131b are also displaced in the Y direction. Due to the movement of the switching member 131, the protruding portion 131a bends the constricting lever 134 in the X direction, and displaces the pressing member 134a in the direction for constricting the first elastic tube 111.

Similarly, due to the movement of the switching member 131, the protruding portion 131b bends the constricting lever 135 in the X direction, and displaces the pressing member 135a in the direction for constricting the second elastic tube 112. In this way, the fluid switching valve 130 switches the fluid introduced to the third elastic tube 113 by constricting or opening each of the first elastic tube 111 and the second elastic tube 112.

The states of the fluid paths in the first elastic tube 111 and the second elastic tube 112 are controlled in association with the position of the switching member 131. As described in detail further below, in the present embodiment, a stepping motor is used as the drive source for driving the switching member 131. Then, the control section of the fluid delivery driving unit 200 controls the position of the switching member 131 with an open loop control system. The control section sets a reference position that is the origin point of the driving as a position at which both the first elastic tube 111 and the second elastic tube 112 are open, and drives the stepping motor from this reference position.

The control section displays fluid path information corresponding to the position of the switching member 131 in the LED display section 208. By looking at the display in the LED display section 208, the user can check whether both the first fluid and second fluid or either one of the first fluid and the second fluid is selected as the fluid or fluids to be discharged.

As described above, a transparent material is used for the top surface of the external packaging member 110. Accordingly, the position of the switching member 131 and the displacement of the constricting lever 134 and the constricting lever 135 can be seen from outside of the external packaging member 110 in a state where the fluid delivery cartridge 100 is mounted on the fluid delivery driving unit 200. The user can also check whether both the first fluid and second fluid or either one of the first fluid and the second fluid is selected as the fluid or fluids to be discharged, by looking at the switching member 131, the constricting lever 134, and the constricting lever 135.

The external packaging member 110 includes an insertion hole 160 near the fluid discharge portion 103 on the inward-facing surface that faces the fluid delivery driving unit 200. A pressure detecting pin is inserted into the insertion hole 160 in order to measure pressure of the third elastic tube 113. Furthermore, the external packaging member 110 includes a cam-housing opening 150 serving as a position fixing member, along with the cam unit 210, at a position opposite the cam unit 210 serving as a position fixing member on the unit side when the external packaging member 110 is mounted on the fluid delivery driving unit 200. The cam-housing opening 150 is a through-hole that contacts the periphery of the cam unit 210.

The external packaging member 110 includes protrusions 115a and 115b, which protrude from the inward-facing surface near two corner regions located on a diagonal of the inward-facing surface that faces the fluid delivery driving unit 200. The protrusions 115a and 115b engage with fixing holes provided in the fluid delivery driving unit 200. In this way, positional misalignment between the fluid delivery cartridge 100 and the fluid delivery driving unit 200 is restricted. Furthermore, the external packaging member 110 includes a through-hole 152. A protruding portion provided on the fluid delivery driving unit 200 engages with the through-hole 152.

The cam unit 210 and the plurality of pressing pins 140 function as a drive mechanism that moves the fluid introduced into the third elastic tube 113 to the fluid discharge portion 103. The plurality of pressing pins 140 are moved between a first position at which the pressing pins 140 constrict the fluid delivery path by pressing the third elastic tube 113 from outside, and a second position at which the pressing pins do not constrict the fluid delivery path. The plurality of pressing pins 140 are arranged along the pin guiding groove 124 formed in the external packaging member 110.

The pressing pins 140 are arranged along the third elastic tube 113 in the delivery direction of the fluid, at uniform intervals in a radial manner centered on the rotational center of the cam unit 210. The tips of the pressing pins 140 protrude toward the cam-housing opening 150 in a direction toward the center of the cam-housing opening 150. Furthermore, the tips of the pressing pins 140 have semi-spherical surfaces oriented in a direction opposite the housing direction of the cam unit 210. By including such semi-spherical surfaces, when the cam unit 210 is housed in the cam-housing opening 150, friction between the tips of the pressing pins 140 and the side surface of the cam body 212 is decreased, and the pressing pins 140 can be arranged reliably at set positions.

Furthermore, when the pressing pins 140 arranged in the pin guiding groove 124 are seen from the outward-facing surface side, the tip portions of the pressing pins 140 are tapered to have a fine tip. In this way, the pressing pins 140 can be arranged radially in a small mounting space. Since the tips of the pressing pins 140 are tapered, the contact surface area between the cam body 212 and the pressing pins 140 is decreased, and the sliding resistance experienced when the cam body 212 is rotationally driven can be decreased.

The cam-housing opening 150 is shaped as an arc centered on the rotational center of the cam unit 210. In this way, when the fluid delivery cartridge 100 is mounted in the fluid delivery driving unit 200, the center of the arc-shaped cam-housing opening 150 can be matched to the rotational center of the cam unit 210. Since the center of the arc-shaped cam-housing opening 150 is matched to the rotational center of the cam unit 210, when the cam unit 210 is rotationally driven while being housed in the cam-housing opening 150, positional misalignment between the fluid delivery cartridge 100 and the cam unit 210 can be prevented.

Furthermore, since the center of the arc-shaped cam-housing opening 150 is matched to the rotational center of the cam unit 210, the pressing pins 140 can sequentially press the third elastic tube 113 with a uniform force in response to the rotational drive of the cam body 212. Accordingly, in response to driving of the cam body 212, the pressing pins 140 can accurately press the third elastic tube 113. As a result, the precise set discharge amount of the fluid can be discharged.

In a state where the fluid delivery cartridge 100 is mounted in the fluid delivery driving unit 200, the pressing pins 140 move along the radial direction according to the driving of the cam body 212. Specifically, the pressing pins 140 move between the first position in which the third elastic tube 113 is pressed from the outside to constrict the fluid delivery path and the second position that does not constrict the fluid delivery path. In this way, the pressing pins 140 deliver fluid from the upstream side to the downstream side inside the third elastic tube 113. In other words, in response to the driving of the cam body 212, the pressing pins 140 sequentially press the third elastic tube 113 from the upstream side to the downstream side, thereby causing the third elastic tube 113 to move peristaltically and deliver the fluid from the upstream side to the downstream side.

On the other hand, when the fluid delivery cartridge 100 is removed from the fluid delivery driving unit 200, i.e. when the cam-housing opening 150 is not housing the cam unit 210, the pressing pins 140 do not press the third elastic tube 113. In other words, when the cam-housing opening 150 is not housing the cam unit 210, the pressing pins 140 are located at the second position in which the elastic force of the third elastic tube 113 causes the fluid delivery path to not be constricted. In this way, when the fluid delivery apparatus 300 is not being used, by removing the fluid delivery cartridge 100 from the fluid delivery driving unit 200, the third elastic tube 113 can be kept in a state of not being pressed upon by any of the pressing pins 140. Accordingly, by maintaining the state in which the third elastic tube 113 is not being pressed upon by any of the pressing pins 140, wear and tear on the third elastic tube 113 can be prevented from progressing.

The cam unit 210, which is the drive mechanism causing the pressing pins 140 to exert pressure, is provided in the fluid delivery driving unit 200. The cam unit 210 desirably rotates accurately in order to accurately discharge the set discharge amount of fluid.

On the other hand, if the cam unit 210 is provided in the fluid delivery cartridge 100, the cam unit 210 is desirably accurately fixed at a predetermined position every time the fluid delivery cartridge 100 is mounted. However, it is difficult to accurately fix the cam unit 210 at the predetermined position. In the present embodiment, by providing the cam unit 210 in the fluid delivery driving unit 200, positional misalignment of the cam unit 210 occurring every time the fluid delivery cartridge 100 is mounted can be prevented. In this way, variations in the fluid discharge amount every time the fluid delivery cartridge 100 is mounted can be prevented.

Furthermore, the fluid delivery cartridge 100 can be attached to and detached from the fluid delivery driving unit 200. Therefore, the fluid delivery cartridge 100 is a consumable product that can be replaced as needed. Accordingly, by replacing the fluid delivery cartridge 100 after a set number of uses, for example, the cleanliness of the fluid delivery cartridge 100 can be maintained.

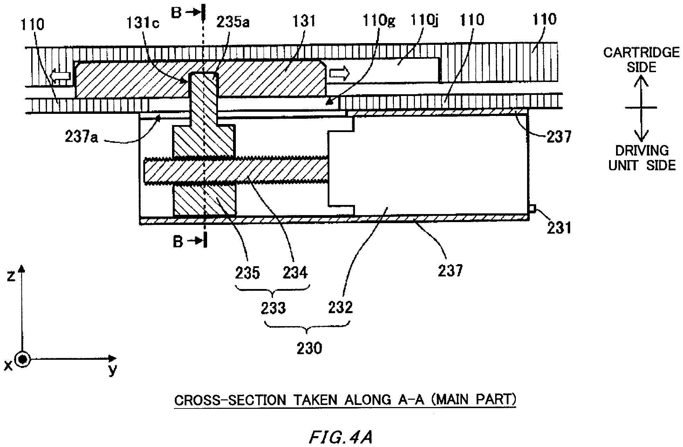

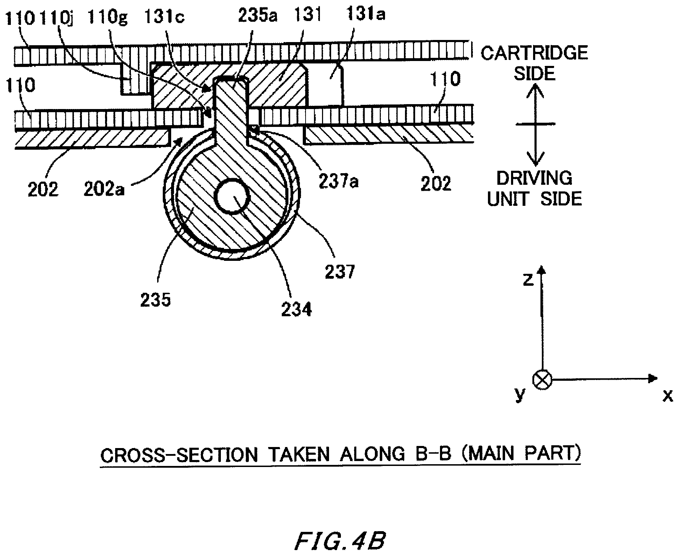

Fig. 4A is a cross-sectional view taken along the line A-A shown in Fig. 3. Fig. 4B is a cross-sectional view taken along the line B-B shown in Fig. 4A. In Figs. 4A and 4B, only the configuration necessary for describing the main portions of the drive mechanism for driving the switching member 131 are extracted and shown.

Figs. 4A and 4B show a state in which the fluid delivery cartridge 100 is mounted in the fluid delivery driving unit 200. The fluid delivery driving unit 200 includes a drive apparatus 230, which is an apparatus for moving the switching member 131 in the Y direction. The drive apparatus 230 includes a motor section 232 and a drive transferring portion 233. The motor section 232 and the drive transferring portion 233 are contained in and supported by a cylindrical support member 237. The support member 237 includes a linear groove 237a.

As shown in Fig. 4B, the mounting surface 202 of the fluid delivery driving unit 200, which is the surface on which the fluid delivery cartridge 100 is mounted, includes a drive transmission opening 202a. The drive apparatus 230 is arranged such that the linear groove 237a of the support member 237 is exposed in the drive transmission opening 202a. The external packaging member 110 is arranged above and in contact with the mounting surface 202, such that the notched groove 110g is above the drive transmission opening 202a.

The motor section 232 generates drive force for moving the switching member 131. The motor section 232 is a stepping motor, as one example. The motor section 232 is electrically connected to a control section and a power source, which are not shown in the drawings, inside the fluid delivery driving unit 200, via the terminal 231. The drive of the motor section 232 is controlled by the control section of the fluid delivery driving unit 200.

The drive transferring portion 233 transfers the drive force generated by the motor section 232 to the switching member 131. The drive transferring portion 233 includes a lead screw 234 and a rack member 235. The lead screw 234 is coupled to an output shaft of the motor section 232, and rotates integrally with the output shaft. A screw thread (male screw) is formed on the front surface of the lead screw 234. The rack member 235 includes a hole through which the lead screw 234 is inserted. A female screw is formed along the surface of this hole, in a manner to engage with the male screw on the surface of the lead screw 234. The female threads in the rack member 235 are meshed with the male threads of the lead screw 234.

The rack member 235 includes a fixing member 235a. The fixing member 235a is inserted into the insertion hole 131c formed in the switching member 131, via the linear groove 237a and the notched groove 110g. In this way, the switching member 131 and the rack member 235 are coupled through the fixing member 235a.

When the lead screw 234 is rotated by the motor section 232, the rack member 235 receives a force causing rotation in the same direction as the rotational direction of the lead screw 234, due to the force of friction at the contact surface between the meshed screws. However, displacement of the fixing member 235a in the rotational direction is limited due to the linear groove 237a. Therefore, the rack member 235 moves translationally in the direction of the rotating shaft of the lead screw 234, i.e. the Y direction, in accordance with the rotation of the lead screw 234. The switching member 131 is locked to the rack member 235 by the fixing member 235a, and therefore the switching member 131 moves in a direction shown by a white arrow in Fig. 4A, according to the translational movement of the rack member 235.

The external packaging member 110 of the fluid delivery cartridge 100 includes a wall portion 110j that prevents reaction. The wall portion 110j is provided in a manner to contact the surface of the switching member 131 that is opposite the side on which the protruding portion 131 a and the protruding portion 131b are provided. When each of the elastic tubes is constricted, the switching member 131 receives a force in a direction opposite the direction causing constriction, due to the repulsive force of the constricting lever 134 or the constricting lever 135. The wall portion 110j restricts displacement of the switching member 131 in the negative X-axis direction caused by the force received from each constricting lever. In this way, the positions of the protruding portion 131a and the protruding portion 131b in the X-axis direction are held at constant positions during the sliding displacement of the switching member 131 in the Y-axis direction.

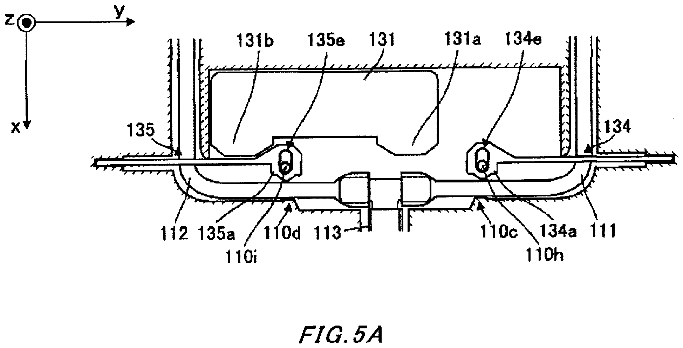

Figs. 5A to 5D are schematic views describing the switching of the flow paths in the fluid switching valve 130. Figs. 5A to 5D are overhead views of the fluid switching valve 130 as seen from above. Fig. 5A shows the fourth state in which neither the first elastic tube 111 nor the second elastic tube 112 is constricted and the first fluid and second fluid are both delivered to the third elastic tube 113. The fourth state is an initial state before the fluid delivery cartridge 100 is used. Before the fluid delivery cartridge 100 is used, the fluid delivery apparatus 300 fixes the fluid switching valve 130 in a manner to hold the flow path state in the fourth state.

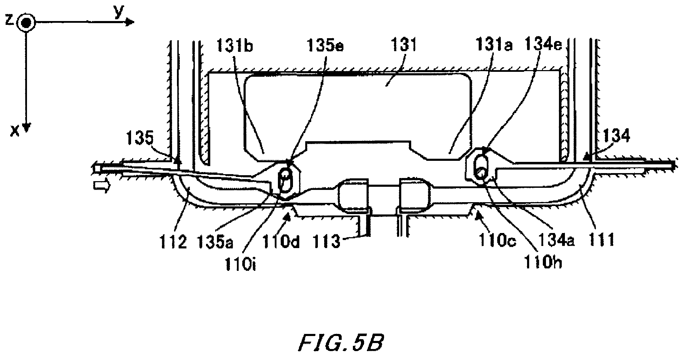

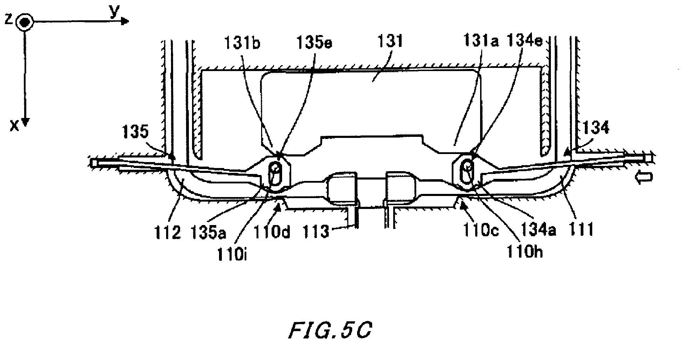

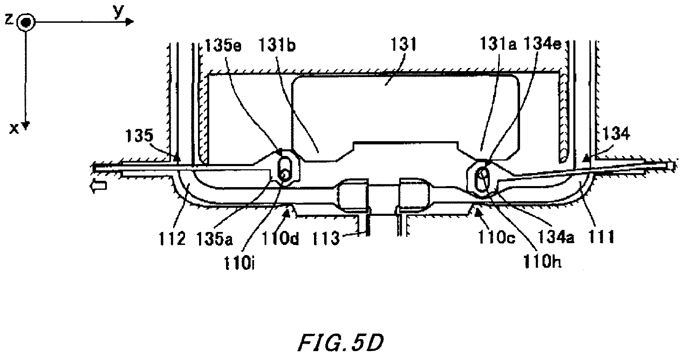

Fig. 5B shows the second state in which the pressing member 135a of the constricting lever 135 constricts the second elastic tube 112 and the first fluid is delivered to the third elastic tube 113. Fig. 5C shows the third state in which the first elastic tube 111 and the second elastic tube 112 are both constricted and the introduction of fluid to the third elastic tube 113 is cut off. At this time, the pressing member 134a of the constricting lever 134 constricts the first elastic tube 111 and the pressing member 135a of the constricting lever 135 constricts the second elastic tube 112. Fig. 5D shows the first state in which the pressing member 134a of the constricting lever 134 constricts the first elastic tube 111 and the second fluid is delivered to the third elastic tube 113.

The switching member 131 slidingly moves in a direction orthogonal to the direction in which the constricting lever 134 and the constricting lever 135 press the first elastic tube 111 and the second elastic tube 112. The switching member 131 slidingly moves in a direction toward the right, in order from Fig. 5A to Fig. 5D. When the switching member 131 moves to the right from the fourth state shown in Fig. 5A, the inclined surface on the right side of the protruding portion 131b of the switching member 131 and the inclined surface on the left side of the pressing member 135a of the constricting lever 135 contact each other.

When the switching member 131 moves farther to the right, the pressing member 135a receives a force and is displaced in the positive X-axis direction, i.e. the direction for constricting the second elastic tube 112, from the switching member 131. More specifically, the displacement of the pressing member 135a in the Y-axis direction is restricted by the restricting pin 110i inserted through the guide hole 135e, and therefore the pressing member 135a is displaced in the positive X-axis direction due to the force received from the protruding portion 131b of the switching member 131. Furthermore, as shown by the white arrow in Fig. 5B, the constricting lever 135 is displaced in the Y-axis direction along with the displacement of the pressing member 135a in the X-axis direction. In this way, the pressing member 135a can press the second elastic tube 112 in a direction substantially orthogonal to the flow path direction.

As shown in Fig. 5B, when the switching member 131 moves until the surface of the protruding portion 131b on the second elastic tube 112 side contacts the surface of the pressing member 135a on the opposite side of the second elastic tube 112, the second elastic tube 112 is constricted by the pressing member 135a. In this way, the flow path of the second fluid is closed and only the first fluid is introduced into the third elastic tube 113.

When the switching member 131 moves right from the second state shown in Fig. 5B, the inclined surface on the right side of the protruding portion 131a of the switching member 131 contacts the inclined surface on the left side of the pressing member 134a of the constricting lever 134. When the switching member 131 moves farther right, the pressing member 134a receives a force and is displaced in the positive X-axis direction, i.e. the direction for constricting the first elastic tube 111, from the switching member 131. More specifically, the displacement of the pressing member 134a in the Y-axis direction is restricted by the restricting pin 110h inserted through the guide hole 134e, and therefore the pressing member 134a is displaced in the positive X-axis direction due to the force received from the protruding portion 131a of the switching member 131. Furthermore, as shown by the white arrow in Fig. 5C, the constricting lever 134 is displaced in the Y-axis direction along with the displacement of the pressing member 134a in the X-axis direction. In this way, the pressing member 134a can press the first elastic tube 111 in a direction substantially orthogonal to the flow path direction.

As shown in Fig. 5C, when the switching member 131 moves until the surface of the protruding portion 131a on the first elastic tube 111 side contacts the surface of the pressing member 134a on the opposite side of the first elastic tube 111, the first elastic tube 111 is constricted by the pressing member 134a. In this way, the flow path for the first fluid is closed. At this time, as shown in Fig. 5C, the second elastic tube 112 is also constricted by the pressing member 135a, and the flow path for the second fluid is also closed. As a result, neither the first fluid nor the second fluid is introduced into the third elastic tube 113.

When the switching member 131 moves to the right from the third state shown in Fig. 5C, the surface of the protruding portion 131b on the second elastic tube 112 side and the surface of the pressing member 135a on the side opposite the second elastic tube 112, which were in contact with each other, move away from each other. Due to the restorative force of the constricting lever 135 in the negative X-axis direction, the pressing member 135a is displaced in the negative X-axis direction, i.e. a direction away from the second elastic tube 112, while the inclined surface on the right side of the pressing member 135a and the inclined surface on the left side of the protruding portion 131b remain in contact with each other.

When the switching member 131 moves farther to the right, the state returns to a state in which the constricting lever 135 completely releases the second elastic tube 112. At this time, as shown in Fig. 5D, the first elastic tube 111 is constricted by the pressing member 134a, and the fluid path for the first fluid is closed. As a result, only the second fluid is introduced into the third elastic tube 113.

When the switching member 131 moves farther right from the first state shown in Fig. 5D, the surface of the protruding portion 131a on the first elastic tube 111 side and the surface of the pressing member 134a on the opposite side of the first elastic tube 111, which were in contact with each other, move away from each other. Due to the restorative force of the constricting lever 134 in the negative X-axis direction, the pressing member 134a is displaced in the negative X-axis direction, i.e. a direction away from the first elastic tube 111, while the inclined surface on the right side of the pressing member 134a and the inclined surface on the left side of the protruding portion 131a remain in contact with each other. At this time, as shown by the white arrow in Fig. 5D, the constricting lever 135 is displaced in the Y-axis direction along with the displacement of the pressing member 135a in the X-axis direction, thereby returning to the position shown in Fig. 5A.

When the switching member 131 moves farther to the right, the state is realized in which the constricting lever 134 completely releases the first elastic tube 111. In this way, the fluid path state becomes the fourth state, in which neither the constricting lever 134 nor the constricting lever 135 is constricting the first elastic tube 111 and the second elastic tube 112.

In the preset embodiment, when the switching member 131 positioned at the left end in the fourth state moves to the right (the positive Y-axis direction), the flow path state switches in the following manner: fourth state → second state → third state → first state → fourth state. Furthermore, when the switching member 131 positioned at the right end in the fourth state moves to the left (the negative Y-axis direction), the flow path state switches in the following manner: fourth state → first state → third state → second state → fourth state.

In the present embodiment, when switching the flow paths between the state in which only the first fluid is introduced into the third elastic tube 113 and the state in which only the second fluid is introduced into the third elastic tube 113, the switching temporarily passes through a state in which both fluid paths are closed. In this way, when switching the fluid paths, it is possible to prevent a mixed fluid including the first fluid and the second fluid from being introduced into the third elastic tube 113.

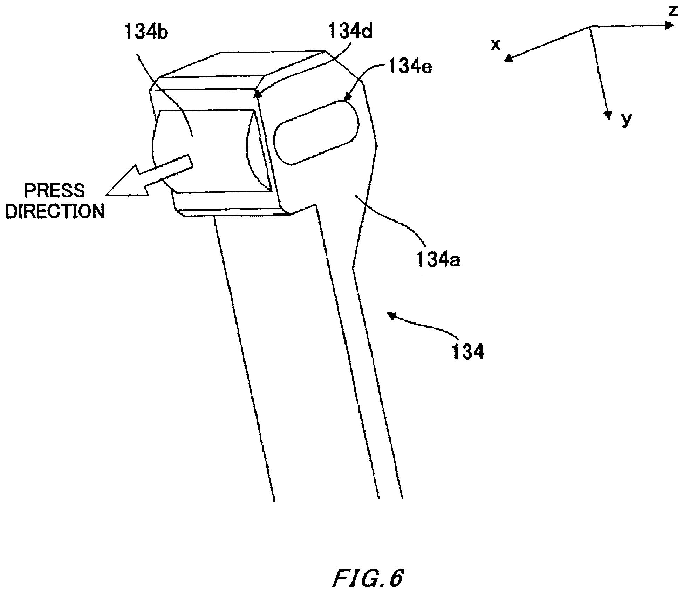

Fig. 6 is a schematic view for describing a protrusion formed on an end portion of a constricting lever. Here, the description uses the constricting lever 134 from among the two constricting levers. The constricting lever 134 includes the protrusion 134b that has less contact surface area on the contact surface 134d with the first elastic tube 111.

As shown in Fig. 6, the protrusion 134b includes a cylindrical surface arranged in a manner to have curvature in the fluid path direction of the first elastic tube 111. By having curvature in the fluid path direction of the first elastic tube 111, the protrusion 134b efficiently constricts the flow path. Furthermore, the width of the protrusion 134b is preferably greater than the width of the first elastic tube 111.

The shape of the surface of the protrusion 134b that contacts the first elastic tube 111 may be a shape other than a cylindrical surface. For example, the shape of the XY cross section may be a portion of a curve other than a circle, such as of an ellipse, a parabola, or a hyperbola. Furthermore, the shape of the entire protrusion 134b does not need to be a portion of a cylinder, and may be a shape such as a portion of a square truncated pyramid or triangular prism arranged such that the surface area on the first elastic tube 111 side is less than the surface are on the contact surface 134d side. By using the protrusion 134b in which the surface area on the first elastic tube 111 side is less than the surface are on the contact surface 134d side, the contact surface area between the pressing member 134a and the first elastic tube 111 becomes smaller, and the pressing member 134a can efficiently constrict the first elastic tube 111.

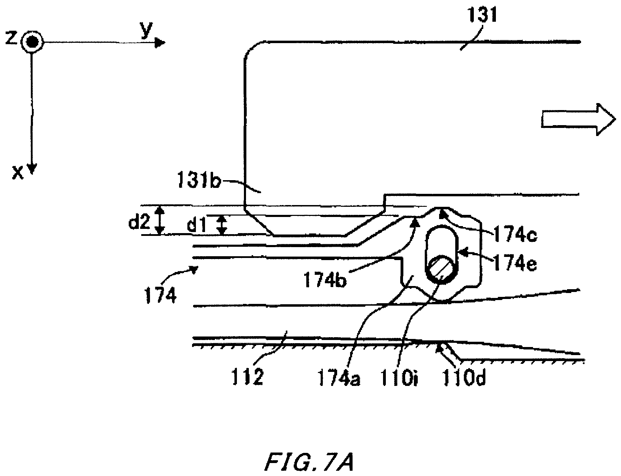

Figs. 7A to 7C are schematic views for describing a constricting lever 174 according to a second embodiment. The same configuration can be adopted on the first elastic tube 111 side and the second elastic tube 112 side, and therefore the description in Figs. 7A to 7C uses the second elastic tube 112 side as a representative configuration.

A first contact portion 174b and a second contact portion 174c are provided in the pressing member 174a of the constricting lever 174, on the contact surface that contacts the switching member 131. The first contact portion 174b and the second contact portion 174c are provided at different positions from each other in the X-axis direction, and form a stepped portion. As shown in Fig. 7A, the length in the X-axis direction from the surface of the protruding portion 131b facing the constricting lever 174 to the first contact portion 174b is d1. Furthermore, the length in the X-axis direction from the surface of the protruding portion 131b facing the constricting lever 174 to the second contact portion 174c is d2. The content already described using Figs. 5A to 5D is omitted here in order to avoid redundancies.

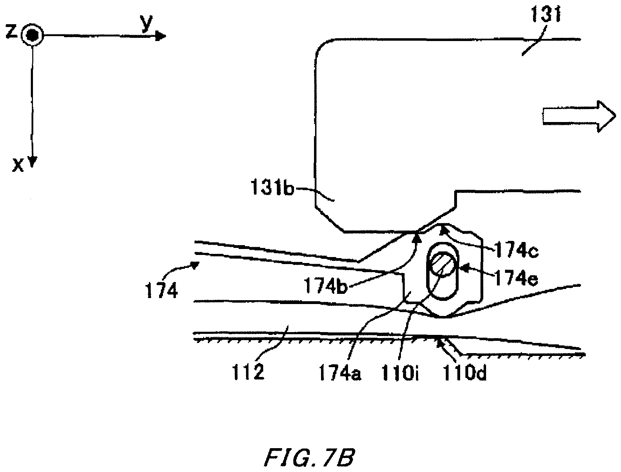

The switching member 131 moves to the right, in order from Fig. 7A to Fig. 7C. As shown in Fig. 7B, when the switching member 131 moves until the surface of the protruding portion 131b on the second elastic tube 112 side contacts the first contact portion 174b of the pressing member 174a, the pressing member 174a is displaced by d1 in the positive X-axis direction. In other words, the pressing member 174a constricts the second elastic tube 112 in an inner radius direction by the displacement amount d1.

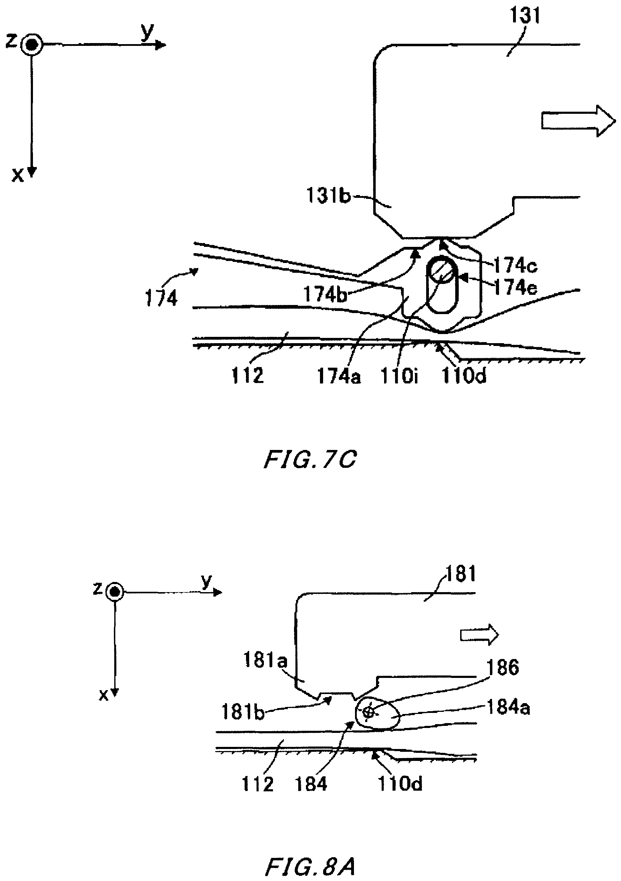

Furthermore, as shown in Fig. 7C, when the switching member 131 moves until the surface of the protruding portion 131b on the second elastic tube 112 side contacts the second contact portion 174c of the pressing member 174a, the pressing member 174a is displaced by d2 in the positive X-axis direction. In other words, the pressing member 174a constricts the second elastic tube 112 in an inner radius direction by the displacement amount d2. In this way, the constricting lever 174 according to the present embodiment can adjust the constriction amount by which the constricting lever 174 constricts the second elastic tube 112 in a stepped manner, by using the stepped portion provided to the pressing member 174a. In this way, it is possible to adjust the introduction amount from the second elastic tube 112 per unit time in a stepped manner.

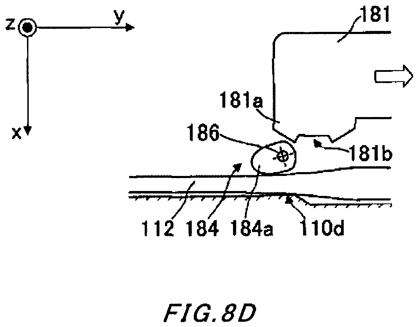

Figs. 8A to 8D are schematic views for describing a constricting mechanism according to a third embodiment. The fluid switching valve according to the present embodiment includes constricting cams 184 instead of the constricting levers of the fluid switching valve 130 according to the first embodiment, and includes a switching member 181 instead of the switching member 131. The same configuration can be adopted on the first elastic tube 111 side and the second elastic tube 112 side, and therefore the description in Figs. 8A to 8C uses the second elastic tube 112 side as a representative configuration.

The constricting cam 184 includes a cam rotation shaft 186 that extends in the Z-axis direction. The constricting cam 184 includes an arch-shaped cam lobe 184a that protrudes from the cam rotation shaft 186 toward the outside. One end of the cam rotation shaft 186 is fixed to the external packaging member 110. The constricting cam 184 is fixed to the other end of the cam rotation shaft 186, in a manner to be rotatable around the center of the cam rotation shaft 186.

The switching member 181 includes a protruding portion 181a and a cam holding hole 181b. The switching member 181 slidingly moves in a direction orthogonal to the direction in which the constricting cam 184 presses the second elastic tube 112. The switching member 181 slidingly moves to the right, in order from Fig. 8A to Fig. 8D. Fig. 8A shows a state in which the constricting cam 184 does not constrict the second elastic tube 112. When the switching member 181 moves to the right, the inclined surface of the protruding portion 181a on the right side contacts the constricting cam 184.

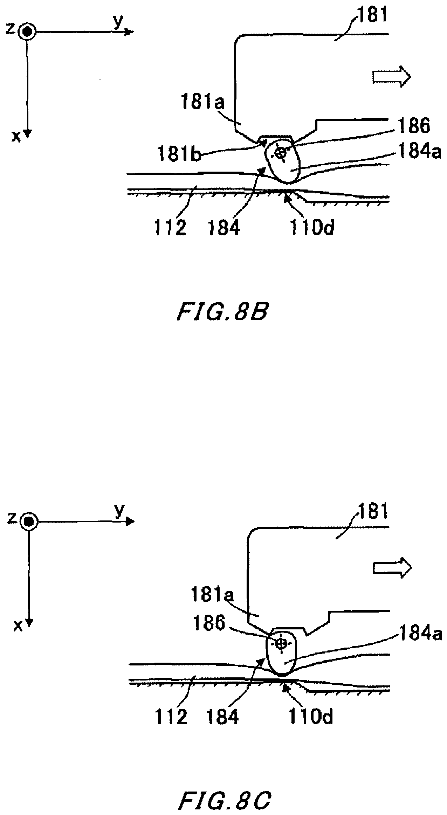

When the switching member 181 moves farther to the right, the constricting cam 184 rotates around the cam rotation shaft 186 due to the force of friction occurring between the constricting cam 184 and the inclined surface of the protruding portion 181a on the right side. As a result of the constricting cam 184 rotating around the cam rotation shaft 186, the cam lobe 184a begins to constrict the second elastic tube 112. When the switching member 181 moves farther to the right, as shown in Fig. 8B, surface on the opposite side of the cam lobe 184a is housed in the cam holding hole 181b at an opposite side of the cam rotation shaft 186 of the constricting cam 184 serving as a base axis. At this time, the constricting cam 184 closes the fluid path of the second elastic tube 112.

When the switching member 181 moves farther to the right from the state shown in Fig. 8B, the surface of the cam holding hole 181b on the left side contacts the constricting cam 184, as shown in Fig. 8C. Then, as a result of the switching member 181 moving farther to the right, the constricting cam 184 receives a force from the surface of the cam holding hole 181b on the left side and rotates around the cam rotation shaft 186. In this way, the position of the cam lobe 184a is shifted and the constricting cam 184 releases the second elastic tube 112 that was being constricted.

The constricting cams in the present embodiment can use components having the same shapes on the first elastic tube 111 side and the second elastic tube 112 side. Therefore, it is possible to reduce the number of types of components, thereby lowering the manufacturing costs.

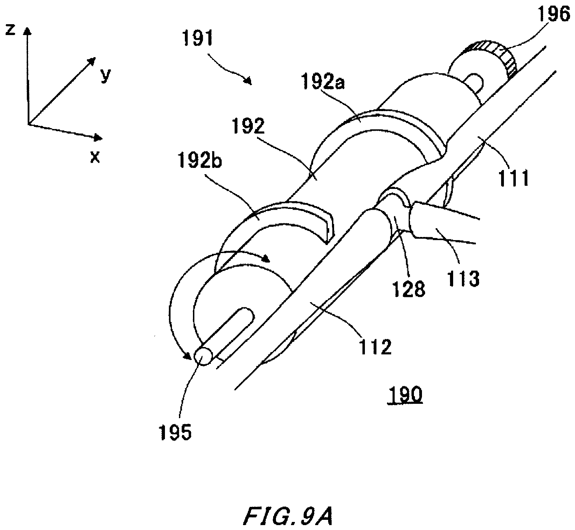

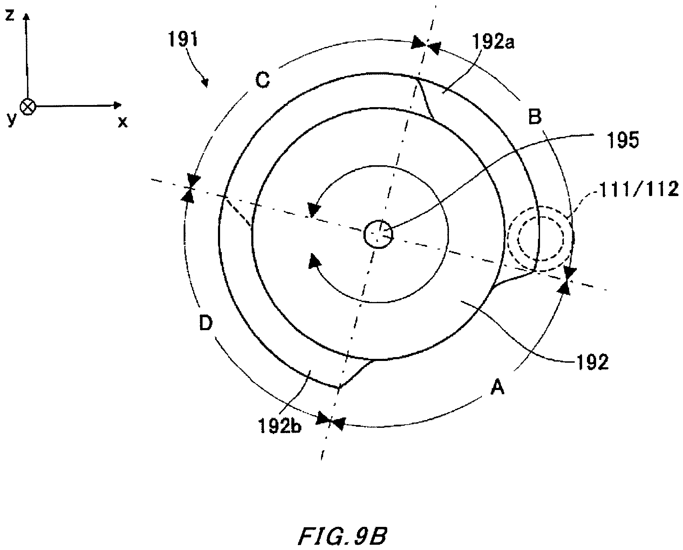

Figs. 9A and 9B are schematic views for describing a fluid switching valve 190 according to a fourth embodiment. Fig. 9A is an external perspective view of the fluid switching valve 190. Fig. 9B shows the fluid switching valve 190 as seen from the negative Y-axis direction.

The fluid switching valve 190 includes the first elastic tube 111, the second elastic tube 112, the third elastic tube 113, the three-way branch member 128, and a switching member 191. Furthermore, the fluid switching valve 190 includes a drive transferring portion 196 serving as a transfer mechanism that transfers drive force generated by an external device such as a stepping motor for rotationally displacing the switching member 191.

The fourth embodiment has a configuration in which the switching member 131, the constricting lever 134, and the constricting lever 135 according to the first embodiment are replaced with the switching member 191. Furthermore, the present embodiment has a configuration in which the drive apparatus 230 according to the first embodiment is replaced with a stepping motor and a gear fixed to the rotating shaft of the stepping motor. In the present embodiment, a portion of the gear fixed to the rotating shaft of the stepping motor is exposed from the mounting surface side of the fluid delivery cartridge 100.

The switching member 191 is a rotating member that moves rotationally. The switching member 191 includes a main body member 192 and a rotating shaft 195. The main body member 192 is a member shaped as a circular pillar, in which a protruding portion 192a that constricts the first elastic tube 111 and a protruding portion 192b that constricts the second elastic tube 112 are formed partially protruding in a radial direction with the rotating shaft 195 as the center, on top of a cylindrical surface.

The protruding portion 192a and the protruding portion 192b are formed at different positions from each other in the Y-axis direction. Furthermore, the protruding portion 192a and the protruding portion 192b are formed in a manner to overlap with each other in a prescribed region, when viewed from the Y-axis direction. A bearing portion, which is not shown in the drawings, for receiving the rotating shaft 195 is provided to the external packaging member 110 of the fluid delivery cartridge 100. The bearing portion fixes the switching member 191 in a manner allowing for rotation, while controlling the displacement of the switching member 191 in the X direction, the Y direction, and the Z direction.

With reference to Fig. 9B, the switching member 191 includes four regions A to D around the circumference, with the rotating shaft 195 as the center. Region A is a region in which neither the protruding portion 192a nor the protruding portion 192b is formed. Region B is a region in which only the protruding portion 192a is formed. Region C is a region in which both the protruding portion 192a and the protruding portion 192b are formed. Region D is a region in which only the protruding portion 192b is formed. In the state shown in Fig. 9B, the first elastic tube 111 is constricted by the protruding portion 192a.

The drive transferring portion 196 is a columnar rotor, and includes a gear in the periphery. The drive transferring portion 196 shares the rotating shaft 195 of the switching member 191. The gear in the periphery of the drive transferring portion 196 engages with the gear fixed to the rotating shaft of the stepping motor, which is not shown in the drawings, and transfers the drive force of this stepping motor to the switching member 191. In other words, the drive transferring portion 196 transfers the rotational force of the stepping motor to the switching member 191 to pivot the switching member 191 around the rotating shaft 195.

When the switching member 191 pivots around the rotating shaft 195, the region that is opposite the first elastic tube 111 and the second elastic tube 112 changes. When region A is opposite the first elastic tube 111 and the second elastic tube 112, the fluid path state is the fourth state, in which neither the first elastic tube 111 nor the second elastic tube 112 is constricted.

When region B is opposite the first elastic tube 111 and the second elastic tube 112, the fluid path state is the first state, in which the first elastic tube 111 is constricted by the protruding portion 192a. When region C is opposite the first elastic tube 111 and the second elastic tube 112, the fluid path state is the third state, in which the first elastic tube 111 is constricted by the protruding portion 192a and the second elastic tube 112 is constricted by the protruding portion 192b. Then, when region D is opposite the first elastic tube 111 and the second elastic tube 112, the fluid path state is the second state, in which the second elastic tube 112 is constricted by the protruding portion 192b.

In the present embodiment, the state in which region A is opposite the first elastic tube 111 and the second elastic tube 112 is the initial state. The control section of the fluid delivery driving unit 200 controls the driving of the stepping motor with an open loop control system, using the position of the initial state as a reference position. When the switching member 191 rotates clockwise as seen from the Y-axis direction, the region opposite the first elastic tube 111 and the second elastic tube 112 changes in the following order: region A → region B → region C → region D → region A. In other words, the switching member 191 switches the fluid path state in the following order: fourth state → first state → third state → second state → fourth state.

Furthermore, when the switching member 191 rotates counter-clockwise as seen from the Y-axis direction, the region opposite the first elastic tube 111 and the second elastic tube 112 changes in the following order: region A → region D → region C → region B → region A. In other words, the switching member 191 switches the fluid path state in the following order: fourth state → second state → third state → first state → fourth state.

In this way, in the present embodiment, when switching the flow paths between the state in which only the first fluid is introduced into the third elastic tube 113 and the state in which only the second fluid is introduced into the third elastic tube 113, the switching temporarily passes through a state in which both fluid paths are closed. In this way, when switching the fluid paths, it is possible to prevent a mixed fluid including the first fluid and the second fluid from being introduced into the third elastic tube 113.

The switching member 191 in the present embodiment can switch the fluid paths by rotating. Accordingly, the switching member 191 does not need space to move in the periphery during the switching of the fluid paths. Therefore, the fluid switching valve can have a more compact configuration.

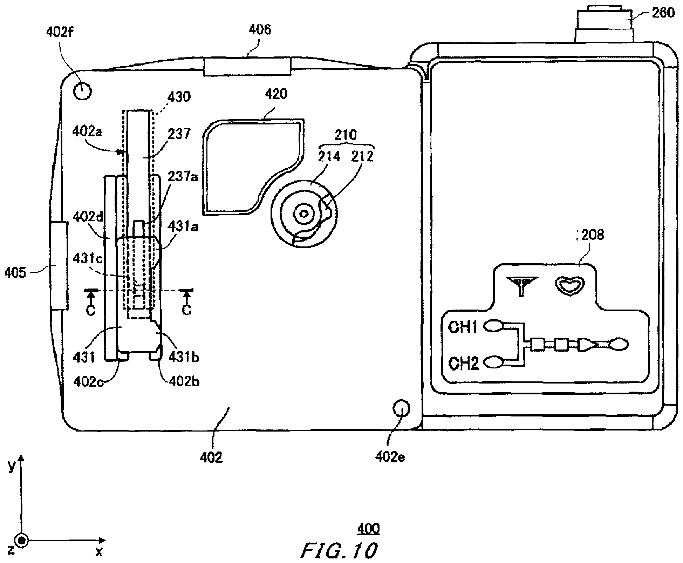

Fig. 10 is a perspective view for describing a fluid delivery driving unit according to a fifth embodiment. In Fig. 2, a state is shown in which the fluid delivery cartridge is mounted in the fluid delivery driving unit, but in the present drawing, only the fluid delivery driving unit is shown. The fluid delivery driving unit 400 according to the present embodiment differs from the fluid delivery driving unit 200 according to the first embodiment, in that a switching member 431 is provided in the fluid delivery driving unit. Components that are the same as components in the first embodiment are given the same reference numerals, and redundant descriptions are omitted.

The fluid delivery driving unit 400 includes the switching member 431 and a drive apparatus 430. The switching member 431 includes a protruding portion 431a, a protruding portion 431b, and an insertion hole 431c. The shapes and functions of the protruding portion 431a and the protruding portion 431 b are the same as those of the protruding portion 131 a and the protruding portion 131b, and therefore descriptions thereof are omitted. Furthermore, the connection between the switching member 431 and the drive apparatus 430 is described further below using the drawings.

A drive transmission opening 402a, which is a rectangular opening, is formed in the mounting surface 402 of the fluid delivery driving unit 400. The fluid delivery driving unit 400 includes a rail portion 402b, a rail portion 402c, and a wall portion 402d. The rail portions 402b and 402c are respectively provided along the long sides of the drive transmission opening 402a. The top surfaces of the rail portions 402b and 402c are formed in substantially the same plane, and each contact the bottom surface of the switching member 431. The switching member 431 slidingly moves in the Y-axis direction along the tops of the rail portions 402b and 402c, due to the drive apparatus 230.

The wall portion 402d is provided in a manner to contact the surface of the switching member 431 that is opposite the side on which the protruding portion 431a and the protruding portion 431b are provided, on the side opposite the drive transmission opening 402a of the rail portion 402c. The wall portion 402d plays the role of a suppressing section that suppresses the rebound from the pressing member of one of the constricting lever 134 and the constricting lever 135. The function of the wall portion 402d is the same as that of the wall portion 110j described using Fig. 4B, and therefore a description thereof is omitted.

A protruding portion 420 with an arc shape relative to the rotational center of the cam unit 210 is provided protruding from the mounting surface 402. Furthermore, a fixing hole 402e and a fixing hole 402f are provided in the mounting surface 402. The protruding portion 420, the fixing hole 402e, and the fixing hole 402f fulfill the role of determining the mounting position of the fluid delivery cartridge when the fluid delivery cartridge is mounted on the mounting surface 402.

The fluid delivery driving unit 400 includes a first hook 405 and a second hook 406, which are described further below, for fixing the fluid delivery cartridge. The functions of the first hook 405 and the second hook 406 are the same as the functions of the first hook 205 and the second hook 206 in the first embodiment, and therefore a description thereof is omitted. In the present embodiment, the mounting surface 402, the protruding portion 420, the fixing hole 402e, and the fixing hole 402f fulfill the role of a mounting portion on which the fluid delivery cartridge is mounted.

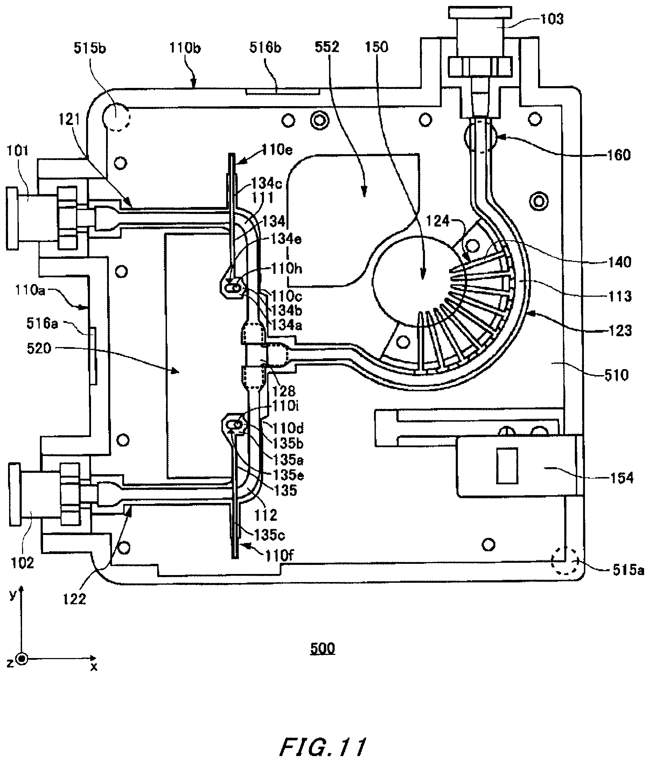

Fig. 11 is a schematic view of the internal configuration of a fluid delivery cartridge according to a fifth embodiment. The fluid delivery cartridge 500 differs from the fluid delivery cartridge 100, in that the fluid delivery cartridge 500 does not include a switching member. Components that are the same as components in the first embodiment are given the same reference numerals, and redundant descriptions are omitted.

The fluid delivery cartridge 500 includes an external packaging member 510. The external packaging member 510 includes a first fastening portion 516a to which the first hook 405 is fixed, on the edge 110a. The external packaging member 510 includes a second fastening portion 516b to which the second hook 406 is fixed, on the edge 110b.

The external packaging member 510 includes an opening 520. The switching member 431 is housed inside the opening 520 when the fluid delivery cartridge 500 is mounted on the fluid delivery driving unit 400.

The external packaging member 510 includes a through-hole 552. The through-hole 552 engages with the protruding portion 420 when the fluid delivery cartridge 500 is mounted on the fluid delivery driving unit 400. Furthermore, the external packaging member 510 includes a protrusion 515a and a protrusion 515b. The protrusion 515a and the protrusion 515b respectively engage with the fixing hole 402e and the fixing hole 402f when the fluid delivery cartridge 500 is mounted on the fluid delivery driving unit 400.

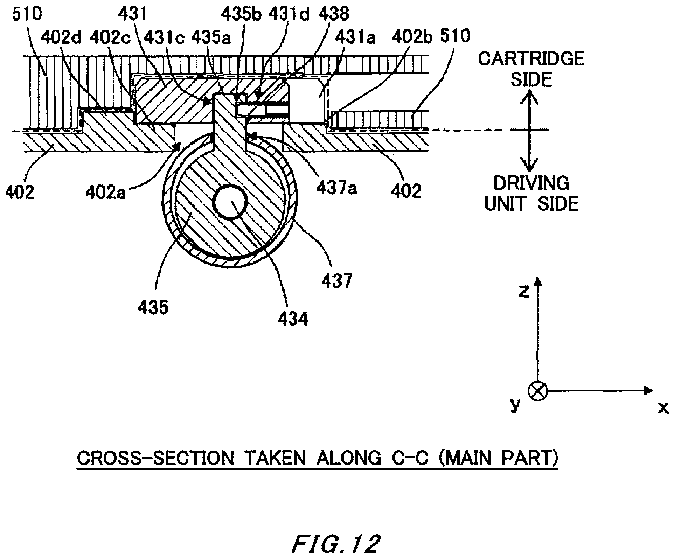

Fig. 12 is a cross-sectional view taken along the line C-C shown in Fig. 10. Fig. 12 shows a state in which the fluid delivery cartridge 500 is mounted on the fluid delivery driving unit 400. Components that are the same as components in the first embodiment are given the same reference numerals, and redundant descriptions are omitted.

The drive apparatus 430 has the same configuration as the drive apparatus 230, aside from the rack member 435. A concave portion 435b is formed in the fixing member 435a of the rack member 435. A screw hole 431d that penetrates through insertion hole 431c from a side surface between the protruding portion 431a and the protruding portion 431b is provided to the switching member 431. A set screw 438 is screwed into the screw hole 431d. The tip portion of the set screw 438 is inserted into the concave portion 435b. By screwing in the set screw 438, the fixing member 435a is pressed against the side surface of the insertion hole 431c, via the concave portion 435b. In this way, the switching member 431 is fixed to the rack member 435 via the set screw 438.

In the fifth embodiment, the protruding portion 431 a and the protruding portion 431b face at least one of the first elastic tube 111 and the second elastic tube 112 when the fluid delivery cartridge 500 is mounted on the mounting portion of the fluid delivery driving unit 400. Then, the fluid path state is switched from the first state to the fourth state, by sliding and displacing the switching member 431 in the Y-axis direction.

In the above description, one example is shown in which a fluid switching valve, for the two flow paths of the first elastic tube 111 and the second elastic tube 112 introducing fluid to the third elastic tube 113, switches the flow paths among four flow path states including combinations of open and closed flow paths. However, the number of flow paths is not limited to two, and there may be three or more flow paths. For example, if there are three flow paths, in Fig. 3, a fourth elastic tube into which fluid is introduced from above is configured to introduce a third fluid. A constricting lever for constricting the fourth elastic tube is provided in a manner similar to the constricting lever 134 and the constricting lever 135. In the present embodiment, the closing and opening of each elastic tube is controlled by using a T-shaped switching member to slidingly move the switching member in the X direction and the Y direction. Similarly, in the case of the fourth flow path, the configuration is such that a fifth elastic tube that has fluid introduced thereto in a direction opposite that of the fourth elastic tube, i.e. introduced from the bottom, is provided to introduce the fourth fluid. In the present embodiment, the closing and opening of each elastic tube is controlled by using a cross-shaped switching member to slidingly move the switching member in the X direction and the Y direction.

In the above description, embodiments are described in which the fluid delivery driving unit includes a drive apparatus that drives the fluid switching valve. However, the fluid delivery cartridge may include the drive apparatus.

In the above description, a stepping motor is used as one example of a component forming the motor section 232 of the drive apparatus 230. However, the component forming the motor section 232 does not need to be a stepping motor. For example, a brushless motor may be used. In this case, a position detecting mechanism for the switching member 131 is provided separately, and feedback control of the drive amount of the motor section 232 may be performed such that the detected position of the switching member 131 becomes a predetermined position. Similarly, for the drive apparatus according to the fourth embodiment as well, the motor section does not need to be a stepping motor, and may be a brushless motor. In this case, a rotary encoder or the like is provided as an angle detection mechanism for the rotating shaft 195, and feedback control of the brushless motor drive amount may be performed using the detected rotational angle of the rotating shaft 195.

In the above description, a drive mechanism including a plurality of pressing pins 140 is used as an example of a drive mechanism that peristaltically moves the third elastic tube 113 to move the fluid therein to the discharge port. However, another drive mechanism may be used. For example, a cam may be arranged relative to the third elastic tube 113 in a manner to change the pressing position and constriction amount of a cam lobe according to rotation, along the direction of the flow path. With such a cam, it is possible to move fluid in the fluid path in a manner to flow from the upstream side to the downstream side. Alternatively, the drive mechanism may be a pump. In this case, the pump is arranged between the end of the third elastic tube 113 and the fluid discharge portion 103, sucks up the fluid in the third elastic tube 113, and expels this fluid from the fluid discharge portion 103.

In the above description, an embodiment in which a stepped portion is provided to the pressing member 174a of the constricting lever 174 is described as one example of an embodiment for adjusting the constriction amount of the elastic tube by the constricting lever 174 in a stepped manner. However, an inclined portion may be provided instead of a stepped portion, and the constriction amount may be adjusted by adjusting the displacement amount of the pressing member 174a according to the positional relationship relative to the switching member 131. Furthermore, the stepped portion may be provided to the protruding portion of the switching member 131 instead of the constricting lever 174. In this case, there is no need to provide the constricting lever with the stepped portion.

In the above description, the fluid switching valve is described as a portion of the configuration of the fluid delivery cartridge 100 in the fluid delivery apparatus 300. However, the fluid switching valve according to the present embodiment can be adopted in various products that include a mechanism for switching fluid flow paths. For example, the fluid switching valve according to the present invention can be adopted in a product that switches between water at different temperatures or switches between fluids (e.g. gas or liquids) in different states.

While the embodiments of the present invention have been described, the technical scope of the invention is not limited to the above described embodiments. It is apparent to persons skilled in the art that various alterations and improvements can be added to the above-described embodiments. It is also apparent from the scope of the claims that the embodiments added with such alterations or improvements can be included in the technical scope of the invention.

The operations, procedures, steps, and stages of each process performed by an apparatus, system, program, and method shown in the claims, embodiments, or diagrams can be performed in any order as long as the order is not indicated by "prior to," "before," or the like and as long as the output from a previous process is not used in a later process. Even if the process flow is described using phrases such as "first" or "next" in the claims, embodiments, or diagrams, it does not necessarily mean that the process must be performed in this order.

## Claims

1. A fluid switching valve comprising:
a first elastic tube that delivers a first fluid;
a second elastic tube that delivers a second fluid;
a third elastic tube having one end connected to the first elastic tube and the second elastic tube;
a switching member that, by displacing a protruding portion, switches between a first state in which the first elastic tube is constricted and the second fluid is delivered to the third elastic tube, a second state in which the second elastic tube is constricted and the first fluid is delivered to the third elastic tube, a third state in which the first elastic tube and the second elastic tube are both constricted and introduction of fluid to the third elastic tube is cut off, and a fourth state in which neither the first elastic tube nor the second elastic tube is constricted and both the first fluid and the second fluid are delivered to the third elastic tube;
a first constricting lever that constricts the first elastic tube; and
a second constricting lever that constricts the second elastic tube, wherein
the protruding portion includes a first protruding portion that presses the first constricting lever to constrict the first elastic tube and a second protruding portion that presses the second constricting lever to constrict the second elastic tube, and
at least one of the first constricting lever and the second constricting lever includes a guide portion for guiding a tip portion thereof toward an elastic tube to be constricted.

2. A fluid switching valve comprising:
a first elastic tube that delivers a first fluid;
a second elastic tube that delivers a second fluid;
a third elastic tube having one end connected to the first elastic tube and the second elastic tube;
a switching member that, by displacing a protruding portion, switches between a first state in which the first elastic tube is constricted and the second fluid is delivered to the third elastic tube, a second state in which the second elastic tube is constricted and the first fluid is delivered to the third elastic tube, a third state in which the first elastic tube and the second elastic tube are both constricted and introduction of fluid to the third elastic tube is cut off, and a fourth state in which neither the first elastic tube nor the second elastic tube is constricted and both the first fluid and the second fluid are delivered to the third elastic tube;
a first constricting lever that constricts the first elastic tube; and
a second constricting lever that constricts the second elastic tube, wherein
the protruding portion includes a first protruding portion that presses the first constricting lever to constrict the first elastic tube and a second protruding portion that presses the second constricting lever to constrict the second elastic tube,
the first constricting lever and the second constricting lever are supported in a manner that enables sliding in a direction parallel to a movement direction of the switching member, at a body portion that extends from a portion pressed by the protruding portion,
the body portion of the first constricting lever includes an escaping portion that crosses over the first elastic tube in a direction orthogonal to a sliding direction, and
the body portion of the second constricting lever includes an escaping portion that crosses over the second elastic tube in a direction orthogonal to the sliding direction.

3. The fluid switching valve according to Claim 1 or 2, wherein
the switching member slidingly moves in a direction orthogonal to a direction in which the first constricting lever and the second constricting lever press the first elastic tube and the second elastic tube.

4. The fluid switching valve according to Claim 1, wherein
at least one of the first constricting lever and the second constricting lever that is provided with the guide portion is supported slidably in one direction.

5. The fluid switching valve according to any one of Claims 1 to 4, wherein the first constricting lever includes a protrusion that has a smaller contact surface area on a contact surface thereof contacting the first elastic tube.

6. The fluid switching valve according to any one of Claims 1 to 5, wherein
a stepped portion is provided on a contact surface of at least one of the first constricting lever and the first protruding portion, and a constriction amount by which the first constricting lever constricts the first elastic tube can be adjusted in a stepped manner.

7. The fluid switching valve according to any one of Claims 1 to 6, wherein
the switching member performs switching in order of the fourth state, the first state, the third state, the second state, and the fourth state, or in order of the fourth state, the second state, the third state, the first state, and the fourth state.

8. A fluid switching valve comprising:
a first elastic tube that delivers a first fluid;
a second elastic tube that delivers a second fluid;
a third elastic tube having one end connected to the first elastic tube and the second elastic tube;
a switching member that, by displacing a protruding portion, switches between a first state in which the first elastic tube is constricted and the second fluid is delivered to the third elastic tube, a second state in which the second elastic tube is constricted and the first fluid is delivered to the third elastic tube, a third state in which the first elastic tube and the second elastic tube are both constricted and introduction of fluid to the third elastic tube is cut off, and a fourth state in which neither the first elastic tube nor the second elastic tube is constricted and both the first fluid and the second fluid are delivered to the third elastic tube;
a first constricting cam that constricts the first elastic tube; and
a second constricting cam that constricts the second elastic tube, wherein
the protruding portion includes a first protruding portion that rotates the first constricting cam to constrict the first elastic tube and a second protruding portion that rotates the second constricting cam to constrict the second elastic tube.

9. The fluid switching valve according to Claim 8, wherein
the switching member slidingly moves in a direction orthogonal to a direction in which the first constricting cam and the second constricting cam press the first elastic tube and the second elastic tube.

10. The fluid switching valve according to any one of Claims 1 to 9, further comprising:
a fourth elastic tube that has one end connected to the third elastic tube and delivers a third fluid; and
a third constricting cam that constricts the fourth elastic tube, wherein
the switching member is T-shaped and controls delivery of the first fluid, the second fluid, and the third fluid by slidingly moving in two directions.

11. The fluid switching valve according to Claim 10, further comprising:
a fifth elastic tube that has one end connected to the third elastic tube and delivers a fourth fluid; and
a fourth constricting cam that constricts the fifth elastic tube, wherein
the switching member is cross-shaped and controls delivery of the first fluid, the second fluid, the third fluid, and the fourth fluid by slidingly moving in two directions.

12. A fluid delivery cartridge comprising:
the fluid switching valve according to any one of Claims 1 to 11;
a first supply port that supplies the first fluid to the first elastic tube;
a second supply port that supplies the second fluid to the second elastic tube; and
a discharge port that discharges the first fluid and the second fluid from the third elastic tube.

13. The fluid delivery cartridge according to Claim 12, wherein
in an initial state before use, the fluid switching valve is fixed to maintain the fourth state.

14. The fluid delivery cartridge according to Claim 12 or 13, comprising:
a transfer mechanism that transfers drive force generated by an external device to displace the switching member.

15. The fluid delivery cartridge according to any one of Claims 12 to 14, comprising:
at least one of a drive mechanism that peristaltically moves the third elastic tube to move fluid therein to the discharge port and a driving section that displaces the switching member.

16. A fluid delivery cartridge that is attachable to and detachable from a fluid delivery driving unit, comprising:
a first elastic tube that delivers a first fluid;
a second elastic tube that delivers a second fluid;
a third elastic tube having one end connected to the first elastic tube and the second elastic tube;
a first constricting lever that constricts the first elastic tube;
a second constricting lever that constricts the second elastic tube;
a first supply port that supplies the first fluid to the first elastic tube;
a second supply port that supplies the second fluid to the second elastic tube; and
a discharge port that discharges the first fluid and the second fluid from the third elastic tube, wherein
at least one of the first constricting lever and the second constricting lever includes a guide portion for guiding a tip portion thereof toward an elastic tube to be constricted, and
when fixed to the fluid delivery driving unit, a switching member of the fluid delivery driving unit switches between a first state in which the first constricting lever is pressed, the first elastic tube is constricted, and the second fluid is delivered to the third elastic tube; a second state in which the second constricting lever is pressed, the second elastic tube is constricted, and the first fluid is delivered to the third elastic tube; a third state in which the first constricting lever and the second constricting lever are pressed, the first elastic tube and the second elastic tube are both constricted, and introduction of fluid to the third elastic tube is cut off; and a fourth state in which neither the first elastic tube nor the second elastic tube is constricted and both the first fluid and the second fluid are delivered to the third elastic tube.

17. A fluid delivery cartridge that is attachable to and detachable from a fluid delivery driving unit, comprising:
a first elastic tube that delivers a first fluid;
a second elastic tube that delivers a second fluid;
a third elastic tube having one end connected to the first elastic tube and the second elastic tube;
a first constricting lever that constricts the first elastic tube;
a second constricting lever that constricts the second elastic tube;
a first supply port that supplies the first fluid to the first elastic tube;
a second supply port that supplies the second fluid to the second elastic tube; and
a discharge port that discharges the first fluid and the second fluid from the third elastic tube, wherein
when fixed to the fluid delivery driving unit, a switching member of the fluid delivery driving unit switches between a first state in which the first constricting lever is pressed, the first elastic tube is constricted, and the second fluid is delivered to the third elastic tube; a second state in which the second constricting lever is pressed, the second elastic tube is constricted, and the first fluid is delivered to the third elastic tube; a third state in which the first constricting lever and the second constricting lever are pressed, the first elastic tube and the second elastic tube are both constricted, and introduction of fluid to the third elastic tube is cut off; and a fourth state in which neither the first elastic tube nor the second elastic tube is constricted and both the first fluid and the second fluid are delivered to the third elastic tube
the first constricting lever and the second constricting lever are supported in a manner that enables sliding in a direction parallel to a movement direction of the switching member, at a body portion that extends from a portion pressed by the protruding portion,
the body portion of the first constricting lever includes an escaping portion that crosses over the first elastic tube in a direction orthogonal to a sliding direction, and
the body portion of the second constricting lever includes an escaping portion that crosses over the second elastic tube in a direction orthogonal to the sliding direction.

18. A fluid delivery driving unit comprising:
a mounting portion on which the fluid delivery cartridge according to any one of Claims 12 to 17 is mounted;
a switching member that includes a protruding portion that faces at least one of the first elastic tube and the second elastic tube when the fluid delivery cartridge is mounted on the mounting portion; and
at least one of a driving section that displaces the switching member and a driving section that displaces a drive mechanism that peristaltically moves the third elastic tube to move fluid therein to the discharge port.

19. The fluid delivery driving unit according to Claim 18, comprising:
a restricting member that restricts reaction of the protruding portion occurring when at least one of the first elastic tube and the second elastic tube is constricted.
